# EUROPÄISCHE PATENTSCHRIFT

(11) **EP 1 537 071 B1**
(45) Veröffentlichungstag und Bekanntmachung des Hinweises auf die Patenterteilung: **05.04.2006**
(21) Anmeldenummer: 03793762.0
(22) Anmeldetag: 28.08.2003
(51) Int. Cl.: C07C 213/00, C07C 29/149

(54) **VERFAHREN ZUR HERSTELLUNG OPTISCH AKTIVER 2-AMINO-,2-CHLOR-,2-HYDROXY- ODER 2-ALKOXY-1-ALKOHOLE**
METHOD FOR THE PRODUCTION OF OPTICALLY ACTIVE 2-AMINO-,2-CHLORO-,2-HYDROXY- OR 2-ALKOXY-1-ALCOHOLS
PROCEDES POUR PRODUIRE DES ALCOOLS OPTIQUEMENT ACTIFS, A SAVOIR DES 2-AMINO-ALCOOLS, 2-CHLORO-ALCOOLS, 2-HYDROXY-ALCOOLS OU 2-ALCOXY-ALCOOLS

(30) Priorität: 04.09.2002 DE 10241292
(43) Veröffentlichungstag der Anmeldung: 08.06.2005
(73) Patentinhaber: BASF Aktiengesellschaft, 67056 Ludwigshafen (DE)
(72) Erfinder: FISCHER, Rolf-Hartmuth, 69121 Heidelberg (DE); BOTTKE, Nils, 68165 Mannheim (DE)
(86) Internationale Anmeldenummer: PCT/EP2003/009513
(87) Internationale Veröffentlichungsnummer: WO 2004/022522

(56) Entgegenhaltungen:
- EP-A- 0 589 168
- EP-A- 1 112 776
- WO-A-98/52891
- WO-A-99/38613
- WO-A-99/38824
- WO-A-99/38838
- DE-A- 2 715 666

## Beschreibung

Die vorliegende Erfindung betrifft ein verbessertes Verfahren zur Herstellung optisch aktiver 2-Amino-, 2-Chlor-, 2-Hydroxy- oder 2-Alkoxy-1-alkohole durch katalytische Hydrierung optisch aktiver 2-Amino-, 2-Chlor-, 2-Hydroxy- oder 2-Alkoxycarbonsäuren oder ihrer Säurederivate.

Wie aus EP-A-696 575 und EP-A-717 023 bekannt, lassen sich optisch aktive 2-Aminocarbonsäuren und 2-Hydroxycarbonsäuren in Gegenwart von Rutheniumkatalysatoren wie elementarem Ruthenium, Rutheniumoxiden und -hydroxiden oder Ruthenium auf Trägern zu optisch aktiven 2-Amino-1-Alkanolen und 1,2-Alkandiolen hydrieren. Bei einer Reaktionsführung zwischen 80 und 100°C bleibt dabei ein Enantiomerenüberschuss von bis zu 98,5 % e.e. erhalten.

Die Ausbeuten und Enantiomerenüberschüsse an 2-Amino-1-alkanolen lassen sich nach WO 99/38838 noch dadurch steigern, dass man die entsprechenden 2-Aminocarbonsäuren in Gegenwart von Mineralsäuren und solchen Rutheniumkatalysatoren hydriert, die ein bis zwei weitere Elemente der Ordnungszahl 23 bis 82 enthalten. Besonders bevorzugt sind dabei Ruthenium/Rhenium-Katalysatoren, bei deren Nutzung Enantiomerenüberschüsse von bis zu 99,9 % e.e. erhalten bleiben.

Nach WO 99/38824 lassen sich auch die Ausbeuten und Enantiomerenüberschüsse an 1,2-Alkandiolen dadurch steigern, dass man Ruthenium-Katalysatoren verwendet, die ein oder zwei weitere Elemente der Ordnungszahlen 23 bis 82 enthalten. Der Zusatz von Rhenium ist dabei besonders bevorzugt.

WO 99/38613 beschreibt ein Verfahren zur Herstellung von besonders vorteilhaften Katalysatoren, die Ruthenium und mindestens ein weiteres Element der Ordnungszahl 23 bis 82 enthalten und deren Verwendung für Hydrierungen. Das Verfahren ist dadurch gekennzeichnet, dass man eine Aufschlämmung einer Rutheniumverbindung, die eine spezifische Oberfläche von 50 bis 300 m²/g aufweist, mit einer Lösung von mindestens einer Metallverbindung zusammenbringt. Besonders bevorzugt sind dabei trägerfreie Ruthenium/Rhenium-Katalysatoren, die für die Herstellung optisch aktiver 2-Aminoalkohole oder 1,2-Diole verwendet werden.

Weiterhin ist bekannt, dass sich optisch aktive 2-Amino- und 2-Hydroxycarbonsäureester bei 25°C und 100 bar Wasserstoffdruck in Gegenwart von aus Rhodium und Platin bestehenden Katalysatoren und einem Lösungsmittel zu entsprechenden optisch aktiven 2-Aminoalkoholen bzw. 1,2-Diolen mit Enantiomerenüberschüssen von über 99 % e.e. hydrieren lassen (M. Studer et al., Adv. Synth. Catal. 2001, 343, Seiten 802-808).

Aus WO 98/52891 ist bekannt, aliphatische Carbonsäuren, Anhydride, Ester oder Lactone in Gegenwart von Platin/RheniumKatalysatoren, die ein weiteres Element wie Molybdän, Silber oder Palladium enthalten, zu den entsprechenden Alkoholen zu hydrieren. Dadurch lassen sich Korrosionsprobleme vermeiden.

Aufgabe der vorliegenden Erfindung ist die Bereitstellung eines verbesserten Verfahrens für die Hydrierung optisch aktiver 2-Amino-, 2-Chlor-, 2-Hydroxy- und 2-Alkoxycarbonsäuren und ihrer Säurederivate zu den entsprechenden optisch aktiven Alkoholen. Die für die Hydrierung zu verwendenden Katalysatoren sollen leicht herstellbar sein, eine hohe Aktivität besitzen und zu hohen Wertproduktausbeuten und Enantiomerenüberschüssen führen.

Diese Aufgabe wird erfindungsgemäß gelöst in einem Verfahren zur Herstellung optisch aktiver 2-Amino-, 2-Chlor-, 2-Hydroxy- oder 2-Alkoxy-1-Alkanole durch katalytische Hydrierung entsprechender optisch aktiver 2-Amino-, 2-Chlor-, 2-Hydroxy- und 2-Alkoxycarbonsäuren oder ihrer Säurederivate, das dadurch gekennzeichnet ist, dass man die Hydrierung in Gegenwart von Palladium und Rhenium oder Platin und Rhenium enthaltenden Katalysatoren durchführt .

In dem erfindungsgemäßen Verfahren kann man z.B. optisch aktive Carbonsäuren oder deren Derivate der Formel I, 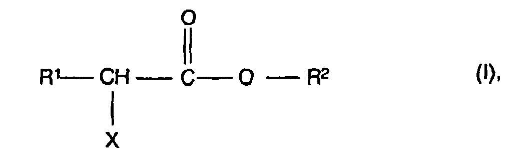 in der die Reste folgende Bedeutung haben:
- R¹:: Geradkettiges oder verzweigtes C₁-C₁₂-Alkyl, C₇-C₁₂-Aralkyl oder C₆-C₁₀-Aryl, wobei die genannten Reste durch NR³R⁴, OH, COOH und/oder weitere, unter den Reaktionsbedingungen stabile Gruppen substituiert sein können,
- R²:: Wasserstoff, geradkettiges oder verzweigtes C₁-C₁₂-Alkyl oder C₃-C₈-Cycloalkyl,
- X:: Chlor, NR⁵R⁶ oder OR⁷,
- R³, R⁴, R⁵ und R⁶:: Unabhängig voneinander jeweils Wasserstoff, geradkettiges oder verzweigtes C₁-C₁₂-Alkyl, C₇-C₁₂-Aralkyl, C₆-C₁₀-Aryl, C₃-C₈-Cycloalkyl oder C₃-C₈-Cycloalkyl, in dem eine CH₂-Gruppe durch 0 oder NR⁸ ersetzt ist,
- R³ und R⁴ sowie R⁵ und R⁶:: Unabhängig voneinander jeweils gemeinsam auch -(CH₂)ₘ-, wobei m eine ganze Zahl von 4 bis 7 bedeutet,
- R¹ und R⁵:: Gemeinsam auch -(CH₂)ₙ-, wobei n einer ganzen Zahl von 2 bis 6 entspricht,
- R⁷:: Wasserstoff, geradkettiges oder verzweigtes C₁-C₁₂-Alkyl oder C₃-C₈-Cycloalkyl,
- R¹ und R⁷ :: Gemeinsam auch -(CH₂)ₙ-, wobei n einer ganzen Zahl von 2 bis 6 entspricht und
- R⁸:: Wasserstoff, geradkettiges oder verzweigtes C₁-C₁₂-Alkyl, C₇-C₁₂-Aralkyl oder C₆-C₁₀-Aryl,
oder deren Säureanhydride einsetzen und zu den entsprechenden optisch aktiven Alkoholen hydrieren.

Die Reste R¹ können breit variiert werden und auch mehrere, z.B. 1 bis 3 unter den Reaktionsbedingungen stabile Substituenten wie NR³R⁴, OH und/oder COOH tragen.

Beispielhaft seien folgende Reste für R¹ genannt:
C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl, C₁-C₁₂-Alkyl wie C₁-C₆-Alkyl (vorstehend genannt) oder unverzweigtes oder verzweigtes Heptyl, Octyl, Nonyl, Decyl, Undecyl oder Dodecyl,
C₇-C₁₂-Aralkyl wie Phenylmethyl, 1-Phenylethyl 2-Phenylethyl, 1-Phenylpropyl, 2-Phenylpropyl oder 3-Phenylpropyl,
C₆-C₁₀-Aryl wie Phenyl, Naphthyl oder Anthracenyl, wobei die aromatischen Reste Substituenten wie NR⁹R¹⁰, OH und/oder COOH tragen können.

Für R² seien beispielhaft folgende Bedeutungen genannt:

Wasserstoff, geradkettiges oder verzweigtes C₁-C₁₂-Alkyl (wie vorstehend genannt) oder C₃-C₈-Cycloalkyl wie z.B. Cyclopropyl, Cyclobutyl, Cyclopentyl, Cyclohexyl, Cycloheptyl und Cyclooctyl.

Anstelle der Carbonsäureester können auch die Säureanhydride als Carbonsäurederivate eingesetzt werden.

Der Rest X steht für Chlor, NR⁵R⁶ oder OR⁷, wobei R⁵ und R⁶, genauso wie R³ und R⁴, bzw. R⁹ und R¹⁰ unabhängig voneinander jeweils für Wasserstoff, geradkettiges oder verzweigtes C₁-C₁₂-Alkyl, insbesondere C₁-C₆-Alkyl, C₇-C₁₂-Aralkyl oder C₆-C₁₀-Aryl, insbesondere Phenyl, oder für C₃-C₈-Cycloalkyl (jeweils wie vorstehend für die Reste R¹ und R² genannt) stehen.

Die Reste R³ und R⁴, R⁵ und R⁶ sowie R⁹ und R¹⁰ können unabhängig voneinander jeweils gemeinsam auch für -(CH₂)ₘ- stehen, wobei m eine ganze Zahl von 4 bis 7, insbesondere 4 oder 5 bedeutet. Dabei kann eine CH₂-Gruppe durch O oder NR⁸ ersetzt sein.

Die Reste R¹ und R⁵ können auch gemeinsam für -(CH₂)ₙ- stehen, wobei n einer ganzen Zahl von 2 bis 6 entspricht.

Der Rest R⁷ steht vorzugsweise für Wasserstoff oder für geradkettiges oder verzweigtes C₁-C₁₂-Alkyl oder C₃-C₈-Cycloalkyl, besonders bevorzugt für Methyl, Ethyl, 1-Methylethyl, 1,1-Dimethylethyl, Hexyl, Cyclohexyl oder Dodecyl. Er kann auch gemeinsam mit R¹ für -(CH₂)ₙ- stehen, wobei n einer ganzen Zahl von 2 bis 6 entspricht.

Durch die erfindungsgemäße Hydrierung werden daraus die entsprechenden optisch aktiven Alkohole der Formel II, 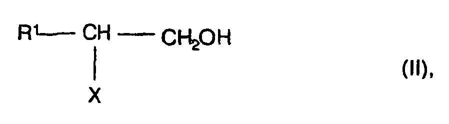 in der R¹ und X die oben genannten Bedeutungen besitzen, erhalten.

Als Ausgangsstoffe kommen beispielsweise 2-Amino-, 2-Chlor-, 2-Hydroxy- oder 2-Alkoxycarbonsäuren und deren Derivate in Betracht, wobei der Rest R¹, soweit unter den Reaktionsbedingungen inert, wie oben beschrieben, breit variiert werden kann.

Aufgrund der leichten Verfügbarkeit werden bevorzugt 2-Aminosäuren der Formel I wie Phenylalanin, Threonin, Glutaminsäure, Prolin, Asparaginsäure, Alanin, Ornithin, Valin, Leucin und Isoleucin und deren Derivate, sowie 2-Hydroxy- und 2-Chlorcarbonsäuren wie Weinsäure, Milchsäure, 2-Chlorpropionsäure und Äpfelsäure und deren Derivate eingesetzt.

Die für das erfindungsgemäße Verfahren verwendeten Katalysatoren enthalten Palladium und Rhenium oder Platin und Rhenium. Sie können mit oder ohne Katalysator-Träger für die erfindungsgemäße Hydrierung verwendet werden. Sie können zusätzlich noch mindestens ein weiteres Element mit einer Ordnungszahl von 23 bis 82 enthalten.

Weitere Elemente in diesem Sinne sind Titan, Vanadium, Chrom, Mangan, Eisen, Kobalt, Nickel, Kupfer, Zink, Zirkon, Molybdän, Silber, Zinn, Wolfram, Blei, Lanthan und Cer, bevorzugt Silber, Wolfram, Molybdän und Zinn, besonders bevorzugt Silber und Zinn.

Das Gewichtsverhältnis von Platin oder Palladium zu Rhenium beträgt vorzugsweise 100:1 bis 0,01:1, besonders bevorzugt 50:1 bis 0,05:1, insbesondere 10:1 bis 0,1:1. Das Gewichtsverhältnis von Platin oder Palladium zu dem mindestens einen weiteren Element beträgt vorzugsweise 100:1 bis 10:1, besonders bevorzugt 50:1 bis 20:1.

Die erfindungsgemäß eingesetzten Katalysatoren können Palladium, Platin, Rhenium und die gegebenenfalls zusätzlichen Elemente in verschiedener Form enthalten, beispielsweise in elementarer Form, in Form von Verbindungen des Palladiums, Platins, Rheniums und der zusätzlichen Elemente oder in Form einer intermetallischen Verbindung des Palladiums, Platins, Rheniums und der zusätzlichen Elemente.

Der Katalysator kann als voll- oder Trägerkatalysator eingesetzt werden. Beim Einsatz als Trägerkatalysator können als Trägermaterial alle geeigneten Materialien, beispielsweise Kohlen, Ruße, Graphite, Siliziumcarbide, Siliziumdioxide, Silikate, Zeolithe, Titandioxid, Zirkondioxid und Tonerden eingesetzt werden. Diese Trägerkatalysatoren können beispielsweise 1 bis 50 Gew.-% Metall in elementarer Form oder in Form von Verbindungen enthalten. Besonders bevorzugt als Trägermaterial ist oxidativ oder mit Mineralsäure vorbehandelte Aktivkohle. Die Herstellung derartiger Katalysatoren ist z.B. in EP-A-848 991 und US 5 698 749 beschrieben.

Falls nicht auf ein Trägermaterial aufgebracht, können die Katalysatoren beispielsweise in kolloidaler Form oder als feinteiliger Feststoff in der erfindungsgemäßen weise eingesetzt werden. Beispiele für Katalysatoren sind fein verteilte Palladium/Rhenium-, Platin/Rhenium-, Palladium/Rhenium/Silber-, Platin/Rhenium/Silber-, Palladium/Rhenium/Molybdän-, Platin/Rhenium/Wolfram-, Platin/Rhenium/Zinn-Partikel, z.B. in metallischer Form oder in Form ihrer Oxide, Hydroxide, Halogenide, Nitrate, Carboxylate, Acetylacetonate oder als Aminkomplexe.

Besonders bevorzugt sind trägerfreie bimetallische Palladium/Rhenium- oder Platin/Rheniumkatalysatoren. Diese können zusätzlich noch mindestens ein weiteres Element der Ordnungszahl 23 bis 82 enthalten. Ihre Herstellung kann z.B. durch Reduktion von Gemischen aus Platinoxid bzw. Palladiumoxid und Rheniumoxid mit einem Reduktionsmittel wie z.B. Wasserstoff erfolgen. Die Abscheidung eines dritten Metalls kann bei der Herstellung des Katalysators oder in situ, während der Hydrierreaktion erfolgen. Die Herstellung solcher Katalysatoren ist beispielsweise in WO 98/52891 beschrieben.

In einer bevorzugten Ausführungsform des erfindungsgemäßen Verfahrens werden die oben beschriebenen optisch aktiven Ausgangsstoffe in Gegenwart einer organischen oder anorganischen Säure hydriert. In der Regel beträgt der Zusatz an Säure 0,5 bis 1,5 Äquivalente, besonders bevorzugt 1 bis 1,3 Äquivalente, bezogen auf 1 Äquivalent der in den Ausgangsstoffen gegebenenfalls vorhandenen basischen Gruppen. Als organische Säuren kommen beispielsweise Essigsäure, Propionsäure und Adipinsäure in Betracht. Bevorzugt ist der Zusatz anorganischer Säuren, insbesondere Schwefelsäure, Salzsäure und Phosphorsäure. Die Säuren können beispielsweise als solche, in Form wässriger Lösungen oder in Form ihrer separat hergestellten Salze mit den zu hydrierenden Ausgangsstoffen, z.B. als Sulfate, Hydrogensulfate, Hydrochloride, Phosphate, Mono- oder Dihydrogenphosphate eingesetzt werden.

Bezogen auf 1 Mol eingesetzter optisch aktiver Ausgangsverbindung kann man z.B. 0,1 bis 10 g der erfindungsgemäß verwendeten, Platin bzw. Palladium, Rhenium und gegebenenfalls zusätzliche Metalle enthaltenden Katalysatoren oder 1 bis 50 g der trägergebundenen Katalysatoren einsetzen.

Im Allgemeinen wird das erfindungsgemäße Verfahren in Gegenwart eines Lösungsmittels für die optisch aktiven Ausgangsstoffe der Formel I durchgeführt. Als Lösungsmittel kommen beispielsweise Wasser, mit Wasser mischbare organische Lösungsmittel und Gemische aus beiden in Frage. Als mit Wasser mischbare Lösungsmittel seien niedere Alkohole mit 1 bis 4 Kohlenstoffatomen und mit Wasser mischbare Ether wie z.B. Tetrahydrofuran oder Dioxan genannt. Bevorzugte Lösungsmittel sind Wasser und Gemische, die Wasser und niedere Alkohole und/oder Tetrahydrofuran enthalten.

Das erfindungsgemäße Verfahren kann man z.B. bei Temperaturen im Bereich von 30 bis 140°C und Drucken im Bereich von 5 bis 300 bar durchführen. Bevorzugt sind Temperaturen von 50 bis 130°C und Drucke von 10 bis 280 bar. Besonders bevorzugt sind Temperaturen von 60 bis 120°C und Drucke von 50 bis 250 bar.

Die Reaktion ist beendet, wenn kein Wasserstoff mehr aufgenommen wird. Üblicherweise beträgt die Hydrierzeit 0,5 bis 8 Stunden.

Zur Aufarbeitung des Reaktionsgemisches kann man beispielsweise zunächst abkühlen, den Katalysator z.B. durch Filtration abtrennen, die vorhandenen leicht flüchtigen Bestandteile wie Lösungsmittel und Reaktionswasser durch Destillation, gegebenenfalls unter vermindertem Druck, teilweise oder ganz entfernen. Im Fall von 2-Aminocarbonsäuren als Ausgangsverbindungen kann man aus dem Rückstand mit Base, z.B. wässriger Alkalilauge oder alkoholischer Alkoholatlösung, den Aminoalkohol aus seinem Salz freisetzen, das ausgefallene Salz abtrennen und das Filtrat im Vakuum fraktionieren. Der abgetrennte Katalysator lässt sich, wie das Lösemittel, wiederverwenden.

Das erfindungsgemäße Verfahren kann kontinuierlich, halb- oder diskontinuierlich durchgeführt werden.

### Ausführungsbeispiele:

### Allgemeine Hydriervorschrift:

In einem Metallautoklaven werden 0,1 g PtO₂ und 0,2 g Re₂O₇, suspendiert in 9 g Wasser, vorgelegt und mit 60 bar Wasserstoff abgepresst. Die Suspension wird 1 Stunde bei 270°C gerührt, nach dem Abkühlen entspannt und 1 g der zu hydrierenden Verbindung hinzugegeben. Danach wird unter den unten angegebenen Bedingungen hydriert.

### Beispiele 1 bis 3: Herstellung von (S)-Leucinol

Analog der angegebenen Vorschrift wurden 1 g enantiomerenreines (L)-Leucin (99,9 % e.e.) zusammen mit 0,5 g konzentrierter Schwefelsäure hydriert. Die Reaktionsbedingungen sind in Tabelle 1 zusammengefasst:

**Tabelle 1**

| Beispiel | Druck [bar] | Temperatur [°C] | Reaktionsdauer [h] |
|---|---|---|---|
| 1 | 100 | 60 | 5 |
| 2 | 100 | 80 | 5 |
| 3 | 100 | 100 | 5 |

Zur Bestimmung der Enantiomerenüberschüsse wurden Proben der Reaktionsausträge mit Natriumhydrogencarbonat neutralisiert, trifluoracetyliert und anschließend gaschromatographisch mittels einer chiralen Cyclodex GTA-Säule analysiert. Die Enantiomerenüberschüsse wurden in allen 3 Beispielen zu größer 99 % e.e. bestimmt.

### Beispiel 4: Herstellung von (S)-1,2-Propandiol

Analog der oben angegebenen Vorschrift wurde 1 g enantiomerenreine (L)-Milchsäure (99,9 % e.e.) 5 Stunden bei 200 bar Wasserstoffdruck und einer Temperatur von 80°C hydriert.

Der Enantiomerenüberschuss des Reaktionsaustrages wurde gaschromatographisch mittels einer Chirasil-Dex-Kapillare zu größer 99 % e.e. bestimmt.

### Beispiel 5: Herstellung von S-1,2,4-Butantriol

In einem Metallautoklaven wurde eine Suspension aus 1,6 g PtO₂ und 4 g Re₂O₇ in 50 g Wasser vorgelegt, mit 60 bar Wasserstoff abgepresst und 1 Stunde bei 270°C und 124 bar gerührt. Nach dem Abkühlen wurde entspannt, 24 g L(-)-Äpfelsäure in 100 ml Wasser zugegeben und anschließend 12 Stunden bei 100°C und einem Druck von 250 bar hydriert. Man erhielt S-1,2,4-Butantriol in einer Ausbeute von 40,8 % und mit einem Enantiomerenüberschuss von 97,2 % e.e.

### Beispiel 6: Herstellung von S-Alaninol

In einem Metallautoklaven wurde eine Suspension aus 0,4 g PtO₂ und 1 g Re₂O₇ in 50 g Wasser vorgelegt, mit 60 bar Wasserstoff abgepresst und 1 Stunde bei 270°C und 125 bar gerührt. Nach dem Abkühlen wurde entspannt, 24 g L-Alanin und 13,8 g konzentrierte Schwefelsäure in 100 ml Wasser zugegeben und anschließend 12 Stunden bei 60°C und einem Druck von 200 bar hydriert. Bei einem Umsatz von 14 % erhielt man Alaninol mit einem Enantiomerenüberschuss von 99,4 % e.e.

### Vergleichsbeispiel 1: Hydrierung von enantiomerenreiner (L)-Milchsäure ohne Re₂O₇

Beispiel 4 wurde unter den angegebenen Reaktionsbedingungen aber unter Weglassen von 0,2 g Re₂O₇ durchgeführt. Die gaschromatographische Analyse ergab, dass sich nur rund 1 % der (L)-Milchsäure zu 1,2-Propandiol umgesetzt hatte.

## Patentansprüche

1. Verfahren zur Herstellung optisch aktiver 2-Amino-, 2-Chlor-, 2-Hydroxy- oder 2-Alkoxy-1-Alkanole durch katalytische Hydrierung entsprechender optisch aktiver 2-Amino-, 2-Chlor-, 2-Hydroxy- und 2-Alkoxycarbonsäuren oder ihrer Säurederivate, **dadurch gekennzeichnet, dass** man die Hydrierung in Gegenwart von Palladium und Rhenium oder Platin und Rhenium enthaltenden Katalysatoren durchführt.

2. Verfahren nach Anspruch 1, **dadurch gekennzeichnet, dass** man optisch aktive 2-Amino-, 2-Chlor, 2-Hydroxy- oder 2-Alkoxycarbonsäuren oder deren Ester der Formel I, 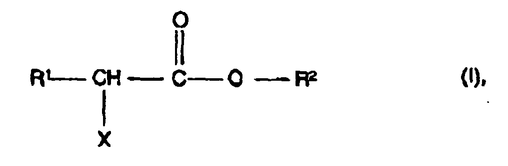 in der die Reste folgende Bedeutung haben:
R¹: Geradkettiges oder verzweigtes C₁-C₁₂ -Alkyl, C₇-C₁₂-Aralkyl oder C₆-C₁₀-Aryl, wobei die genannten Reste durch NR³R⁴, OH, COOH und/oder weitere, unter den Reaktionsbedingungen stabile Gruppen substituiert sein können,
R²: Wasserstoff, geradkettiges oder verzweigtes C₁-C₁₂-Alkyl oder C₃-C₈-Cycloalkyl,
X: Chlor, NR⁵R⁶ oder OR⁷,
R³, R⁴, R⁵ und R⁶: Unabhängig voneinander jeweils Wasserstoff, geradkettiges oder verzweigtes C₁-C₁₂-Alkyl, C₇-C₁₂-Aralkyl, C₆-C₁₀-Aryl, C₃-C₈-Cycloalkyl oder C₃-C₈-Cycloalkyl, in dem eine CH₂-Gruppe durch 0 oder NR⁸ ersetzt ist.
R³ und R⁴ sowie R⁵ und R⁶: Unabhängig voneinander jeweils gemeinsam auch -(CH₂)ₘ-, wobei m eine ganze Zahl von 4 bis 7 bedeutet,
R¹ und R⁵: Gemeinsam auch -(CH₂)ₙ-, wobei n einer ganzen Zahl von 2 bis 6 entspricht,
R⁷. Wasserstoff, geradkettiges oder verzweigtes C₁-C₁₂-Alkyl oder C₃-C₈-Cycloalkyl,
R¹ und R⁷: Gemeinsam auch -(CH₂)ₙ- , wobei n einer ganzen Zahl von 2 bis 6 entspricht und
R⁸: Wasserstoff, geradkettiges oder verzweigtes C₁-C₁₂-Alkyl, C₇-C₁₂-Aralkyl oder C₆-C₁₀-Aryl,
oder deren Säureanhydride einsetzt und zu den entsprechenden optisch aktiven Alkoholen der Formel II 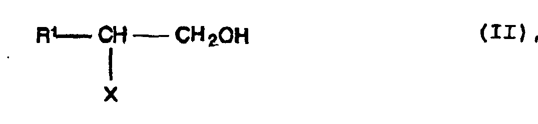 in der R¹ und X die oben genannten Bedeutungen besitzen, hydriert.

3. Verfahren nach den Ansprüchen 1 und 2, **dadurch gekennzeichnet, dass** die Palladium/Rhenium- oder Platin/RheniumKatalysatoren mindestens ein Element aus der Gruppe der Elemente Titan, Vanadium, Chrom, Mangan, Eisen, Kobalt, Nickel, Kupfer, Zink, Zirkon, Molybdän, Silber, Zinn, Wolfram, Blei, Lanthan und Cer enthalten.

4. Verfahren nach den Ansprüchen 1 und 2, **dadurch gekennzeichnet, dass** die Palladium/Rhenium- oder Platin/RheniumKatalysatoren mindestens ein Element aus der Gruppe der Elemente Silber, Molybdän, Wolfram oder Zinn enthalten.

5. Verfahren nach den Ansprüchen 1 bis 4, **dadurch gekennzeichnet, dass** die Palladium/Rhenium- oder Platin/RheniumKatalysatoren ungeträgert oder auf einen Träger aufgebracht eingesetzt werden.

6. Verfahren nach den Ansprüchen 1 bis 5, **dadurch gekennzeichnet, dass** das Gewichtsverhältnis der Elemente Palladium oder Platin zu Rhenium 100:1 bis 0,01;1 beträgt.

7. Verfahren nach den Ansprüchen 1 bis 6, **dadurch gekennzeichnet, dass** das Gewichtsverhältnis der Elemente Palladium oder Platin zu Rhenium 50:1 bis 0,05:1 beträgt.

8. Verfahren nach den Ansprüchen 1 bis 7, **dadurch gekennzeichnet, dass** das Gewichtsverhältnis der Elemente Palladium oder Platin zu dem mindestens einen weiteren Element des Katalysators 100:1 bis 10:1 beträgt.

9. Verfahren nach den Ansprüchen 1 bis 8, **dadurch gekennzeichnet, dass** man die Hydrierung in Gegenwart einer Säure durchführt.

10. Verfahren nach den Ansprüchen 1 bis 9, **dadurch gekennzeichnet, dass** man die Hydrierung bei einer Temperatur von 30 bis 140°C durchführt.

## Claims

1. A process for preparing optically active 2-amino-, 2-chloro-, 2-hydroxy- or 2-alkoxy-1-alkanols by catalytically hydrogenating appropriate optically active 2-amino-, 2-chloro-, 2-hydroxy- and 2-alkoxycarboxylic acids or their acid derivatives, which comprises carrying out the hydrogenation in the presence of catalysts comprising palladium and rhenium or platinum and rhenium.

2. The process according to claim 1, wherein optically active 2-amino-, 2-chloro-, 2-hydroxy- or 2-alkoxycarboxylic acids or their esters of the formula I 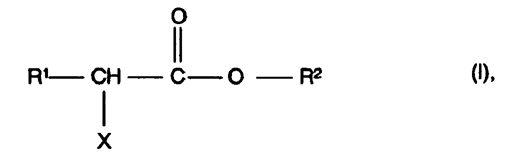 where the radicals are defined as follows:
R¹: straight-chain or branched C₁-C₁₂-alkyl, C₇-C₁₂-aralkyl or C₆-C₁₀-aryl, each of which may be substituted by NR³R⁴, OH, COOH and/or further groups stable under the reaction conditions,
R²: hydrogen, straight-chain or branched C₁-C₁₂-alkyl or C₃-C₈-cycloalkyl,
X: chlorine, NR⁵R⁶ or OR⁷,
R³, R⁴, R⁵ and R⁶: each independently hydrogen, straight-chain or branched C₁-C₁₂-alkyl, C₇-C₁₂-aralkyl, C₆-C₁₀-aryl, C₃-C₈-cycloalkyl or C₃-C₈-cycloalkyl in which one CH₂ group is replaced by O or NR⁸,
R³ and R⁴ and also R⁵ and R⁶: also each independently together -(CH₂)ₘ-, where m is an integer from 4 to 7,
R¹ and R⁵: also together -(CH₂)ₙ- where n is an integer from 2 to 6,
R⁷: hydrogen, straight-chain or branched C₁-C₁₂-alkyl or C₃-C₈-cycloalkyl,
R¹ and R⁷: also together -(CH₂)ₙ-, where n is an integer from 2 to 6 and
R⁸: hydrogen, straight-chain or branched C₁-C₁₂-alkyl, C₇-C₁₂-aralkyl or C₆-C₁₀-aryl,
or their acid anhydrides are used and hydrogenated to the corresponding optically active alcohols of the formula II 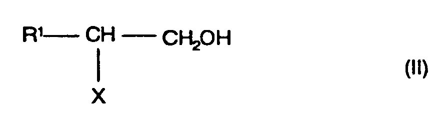 in which R¹ and X are each as defined above.

3. The process according to claims 1 and 2, wherein the palladium/rhenium or platinum/rhenium catalysts comprise at least one element from the group of the elements titanium, vanadium, chromium, manganese, iron, cobalt, nickel, copper, zinc, zirconium, molybdenum, silver, tin, tungsten, lead, lanthanum and cerium.

4. The process according to claims 1 and 2, wherein the palladium/rhenium or platinum/rhenium catalysts comprise at least one element from the group of the elements silver, molybdenum, tungsten and tin.

5. The process according to any of claims 1 to 4, wherein the palladium/rhenium or platinum/rhenium catalysts are used unsupported or applied to a support.

6. The process according to any of claims 1 to 5, wherein the weight ratio of the elements palladium or platinum to rhenium is from 100:1 to 0.01:1.

7. The process according to any of claims 1 to 6, wherein the weight ratio of the elements palladium or platinum to rhenium is from 50:1 to 0.05:1.

8. The process according to any of claims 1 to 7, wherein the weight ratio of the elements palladium or platinum to the at least one further element of the catalyst is from 100:1 to 10:1.

9. The process according to any of claims 1 to 8, wherein the hydrogenation is carried out in the presence of an acid..

10. The process according to any of claims 1 to 9, wherein the hydrogenation is carried out at a temperature of from 30 to 140°C.

## Revendications

1. Procédé de préparation de 2-amino-1-alcanols, de 2-chloro-1-alcanols, de 2-hydroxy-1-alcanols ou de 2-alcoxy-1-alcanols optiquement actifs par hydrogénation catalytique d'acides 2-aminocarboxyliques, 2-chlorocarboxyliques, 2-hydroxycarboxyliques et 2-alcoxycarboxyliques optiquement actifs correspondants ou de leurs dérivés d'acide, **caractérisé en ce qu'**on effectue l'hydrogénation en présence de catalyseurs contenant du palladium et du rhénium ou du platine et du rhénium.

2. Procédé suivant la revendication 1, **caractérisé en ce qu'**on met en oeuvre des acides 2-aminocarboxyliques, 2-chlorocarboxyliques, 2-hydroxycarboxyliques ou 2-alcoxycarboxyliques optiquement actifs ou leurs esters de la formule I 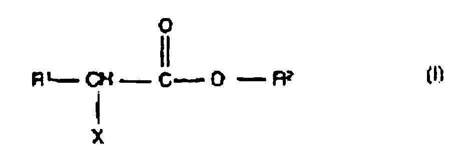 dans laquelle les radicaux ont la signification suivante :
R¹ représente un groupe alkyle en C₁-C₁₂ linéaire ou ramifié, aralkyle en C₇-C₁₂ ou aryle en C₆-C₁₀, les radicaux cités pouvant être substitués par NR³R⁴, OH, COOH et/ou d'autres groupes stables dans les conditions réactionnelles,
R² représente de l'hydrogène ou un groupe alkyle en C₁-C₁₂ linéaire ou ramifié ou cycloalkyle en C₃-C₈,
X représente du chlore, NR⁵R⁶ ou OR⁷,
R³, R⁴, R⁵ et R⁶ représentent, indépendamment l'un de l'autre, de l'hydrogène ou un groupe alkyle en C₁-C₁₂ linéaire ou ramifié, aralkyle en C₇-C₁₂, aryle en C₆-C₁₀, cycloalkyle en C₃-C₈ ou cycloalkyle en C₃-C₈ dans lequel un groupe CH₂ est remplacé par O ou NR⁸,
R³ et R⁴ ainsi que R⁵ et R⁶ pouvant, indépendamment l'un de l'autre, représenter conjointement également -(CH₂)ₘ-, où m représente un nombre entier de 4 à 7,
R¹ et R⁵ pouvant aussi représenter conjointement -(CH₂)ₙ-, où n correspond à un nombre entier de 2 à 6,
R⁷ représente de l'hydrogène ou un groupe alkyle en C₁-C₁₂ linéaire ou ramifié ou cycloalkyle en C₃-C₈,
R¹ et R⁷ pouvant aussi représenter conjointement -(CH₂)ₙ-, où n correspond à un nombre entier de 2 à 6, et
R⁸ représente de l'hydrogène ou un groupe alkyle en C₁-C₁₂ linéaire ou ramifié, aralkyle en C₇-C₁₂ ou aryle en C₆-C₁₀,
ou leurs anhydrides et on les hydrogène en les alcools optiquement actifs correspondants de la formule II 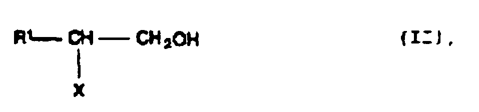 dans laquelle R¹ et X ont les significations indiquées ci-dessus.

3. Procédé suivant les revendications 1 et 2, **caractérisé en ce que** les catalyseurs à base de palladium/rhénium ou de platine/rhénium contiennent au moins un élément du groupe des éléments titane, vanadium, chrome, manganèse, fer, cobalt, nickel, cuivre, zinc, zirconium, molybdène, argent, étain, tungstène, plomb, lanthane et cérium.

4. Procédé suivant les revendications 1 et 2, **caractérisé en ce que** les catalyseurs à base de palladium/rhénium ou de platine/rhénium contiennent au moins un élément du groupe des éléments argent, molybdène, tungstène ou étain.

5. Procédé suivant les revendications 1 à 4, **caractérisé en ce que** les catalyseurs à base de palladium/rhénium ou de platine/rhénium sont mis en oeuvre sans support ou appliqués sur un support.

6. Procédé suivant les revendications 1 à 5, **caractérisé en ce que** le rapport pondéral entre les éléments palladium ou platine et rhénium est de 100/1 à 0,01/1.

7. Procédé suivant les revendications 1 à 6, **caractérisé en ce que** le rapport pondéral entre les éléments palladium ou platine et rhénium est de 50/1 à 0,05/1.

8. Procédé suivant les revendications 1 à 7, **caractérisé en ce que** le rapport pondéral entre les éléments palladium ou platine et le au moins un autre élément du catalyseur est de 100/1 à 10/1.

9. Procédé suivant les revendications 1 à 8, **caractérisé en ce que** l'on effectue l'hydrogénation en présence d'un acide.

10. Procédé suivant les revendications 1 à 9, **caractérisé en ce qu'**on effectue l'hydrogénation à une température de 30 à 140°C.
